(19) Europäisches Patentamt / European Patent Office / Office européen des brevets

(11) **EP 3 745 124 A1**

(12) **EUROPEAN PATENT APPLICATION**
published in accordance with Art. 153(4) EPC

(43) Date of publication:
**02.12.2020 Bulletin 2020/49**

(51) Int Cl.:
*G01N 29/02* (2006.01)    *G01N 5/02* (2006.01)
*G01N 27/00* (2006.01)

(21) Application number: **19743371.7**

(22) Date of filing: **23.01.2019**

(86) International application number:
**PCT/JP2019/002145**

(87) International publication number:
**WO 2019/146662 (01.08.2019 Gazette 2019/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.01.2018 JP 2018008758**
**31.01.2018 JP 2018016030**

(71) Applicant: **Kyocera Corporation**
**Fushimi-ku, Kyoto-shi,**
**Kyoto 612-8501 (JP)**

(72) Inventors:
• **KURIOKA, Hideharu**
**Kyoto-shi, Kyoto 612-8501 (JP)**
• **TANAKA, Hiroyasu**
**Kyoto-shi, Kyoto 612-8501 (JP)**

(74) Representative: **Viering, Jentschura & Partner mbB**
**Patent- und Rechtsanwälte**
**Am Brauhaus 8**
**01099 Dresden (DE)**

(54) **MEASUREMENT METHOD AND MEASUREMENT DEVICE**

(57)    A measurement method according to one embodiment, includes: a preparation step (A1) of preparing a detecting section on an upper surface of which a reaction substance that can react specifically with a detection substance and a correction substance is placed and which can measure a signal value based on a reaction of the reaction substance; a first supply step (A21) of supplying the detection substance onto the detecting section; a second supply step (A22) of supplying the correction substance onto the detecting section; a first measurement step (A3) of measuring a first signal value based on a specific reaction between the detection substance and the reaction substance; a second measurement step (A4) of measuring a second signal value based on a specific reaction between the correction substance and the reaction substance; and a correction step (A5) of correcting the first signal value with use of the second signal value.

*FIG. 1*

**Description**

Technical Field

The present disclosure relates to a measurement method and a measurement device.

Background Art

There are known methods and devices for measurement on substances contained in a sample by means of ELISA using antigen-antibody reaction.

Recently, a measurement method for measure substances in a sample using a biosensor including a detecting element with an antibody bound to a surface of the detecting element.

Summary of Invention

A measurement method according to an embodiment includes: preparing a detecting section on an upper surface of a measurement device with a reaction substance that can react specifically with a detection substance and a correction substance, wherein the detecting section measures signal values based on reactions of the reaction substance; supplying the detection substance onto the detecting section; supplying the correction substance onto the detecting section; measuring a first signal value of the signal values based on a specific reaction between the detection substance and the reaction substance; measuring a second signal value of the signal values based on a specific reaction between the correction substance and the reaction substance; and correcting the first signal value using the second signal value.

A measurement method according to an embodiment includes: a first reaction step of reacting a detection substance with a reaction substance which reacts specifically with each of the detection substance and a correction substance; a second reaction step of reacting the correction substance with the reaction substance; a first measurement step of measuring a first signal value derived from a reaction between the detection substance and the reaction substance; a second measurement step of measuring a second signal value derived from a reaction between the correction substance and the reaction substance; and a correction step of correcting the first signal value with use of the second signal value. The second reaction step and the second measurement step are carried out prior to the first reaction step or subsequent to the first measurement step.

A measurement method according to an embodiment includes: a first reaction step of reacting a detection substance with a first reaction substance which reacts specifically with the detection substance; a second reaction step of reacting a correction substance with a second reaction substance which specifically reacts at least with the correction substance; a first measurement step of measuring a first signal value derived from a reaction between the detection substance and the first reaction substance; a second measurement step of measuring a second signal value derived from a reaction between the second reaction substance and the correction substance; and a correction step of correcting the first signal value with use of the second signal value.

A measurement device according to an embodiment includes: a detecting section on an upper surface of the measurement device on which a reaction substance that can react specifically with a detection substance and a correction substance is placed, and which can measure signal values based on reactions of the reaction substance; a supply section which supplies a detection substance and a correction substance to the detecting section; a measurement section which measures a first signal value of the signal values based on a reaction between the detection substance and the reaction substance, and a second signal value of the signal values based on a reaction between the correction substance and the reaction substance; and a correction section which corrects the first signal value using the second signal value.

A measurement device according to an embodiment includes: a first reaction section which reacts a detection substance with a first reaction substance which reacts specifically with the detection substance; a second reaction section which reacts a correction substance with a second reaction substance which specifically reacts at least with the correction substance; a measurement section which measures a first signal value derived from a reaction between the detection substance and the first reaction substance, and a second signal value derived from a reaction between the correction substance and the second reaction substance; and a correction section which corrects the first signal value with use of the second signal value.

Brief Description of Drawings

FIG. 1 is a flow chart for a measurement method according to one embodiment;

FIG. 2 is a flow chart for a modified example of the measurement method according to one embodiment;

FIG. 3 is a diagram schematically showing an example of one embodiment;

FIG. 4 is a diagram schematically showing an example of one embodiment;

FIG. 5 is a flow chart for a measurement method according to another embodiment;

FIG. 6 is a flow chart for a modified example of the measurement method according to another embodiment;

FIG. 7 is a diagram schematically showing an example of another embodiment;

FIG. 8 is a diagram schematically showing an example of another embodiment;

FIG. 9 is a diagram schematically showing an example of another embodiment;

FIG. 10 is a diagram schematically showing an example of another embodiment;

FIG. 11 is a diagram schematically showing an example of another embodiment;

FIG. 12 is a drawing showing the configuration of a measurement device according to one embodiment;

FIG. 13 is a perspective view of a biosensor device which is an example of the measurement device according to one embodiment;

FIG. 14 is an exploded perspective view of the biosensor device which is an example of the measurement device according to one embodiment;

FIG. 15 is a plan view of a detecting element used in the measurement device according to one embodiment; and

FIG. 16 is a drawing showing the configuration of a modified example of the measurement device according to another embodiment.

Description of Embodiments

<Measurement Method>

(One Embodiment)

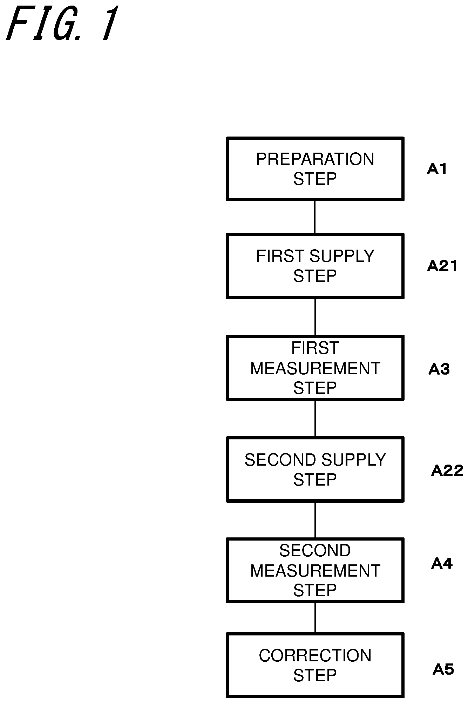

FIG. 1 is a flow chart for a measurement method according to one embodiment.

The measurement method according to the embodiment includes:

a preparation step A1 of preparing a detecting section on an upper surface of which a reaction substance that can react specifically with a detection substance and a correction substance is placed and which can measure a signal value based on a reaction of the reaction substance;

a first supply step A21 of supplying the detection substance onto the detecting section;

a second supply step A22 of supplying the correction substance onto the detecting section;

a first measurement step A3 of measuring a first signal value based on a specific reaction between the detection substance and the reaction substance;

a second measurement step A4 of measuring a second signal value based on a specific reaction between the correction substance and the reaction substance; and

a correction step A5 of correcting the first signal value with use of the second signal value.

In the measurement method according to the above-described embodiment,

the reaction substance may react specifically with each of the detection substance and the correction substance, and the second supply step A22 and the second measurement step A4 may be carried out prior to the first supply step A211 or subsequent to the first measurement step A23.

As a problem associated with the related art, in order to detect a substance contained in a sample with higher sensitivity, it takes much operation time and high cost. As another problem, due to variations in sensitivity between the detecting sections, calibration needs to be done every time each measuring operation is carried out, which results in increased complexity of operations.

In this regard, the measurement method according to one embodiment configured as described above allows correction for variations in sensitivity between detecting sections, and thus achieves highly accurate measurement on substances contained in a sample.

The measurement method according to the embodiment will now be described in a step-by-step manner.

The preparation step A1 is intended to prepare a detecting section capable of measuring a signal value based on a reaction between a reaction substance placed on the upper surface of the detecting section and each of a detection substance and a correction substance.

For example, the "detecting section" includes, on an upper surface thereof, a detecting element which produces signal value output. Examples of the detecting element may include, but is not limited to, an elastic wave device, a QCM (Quartz Crystal Microbalance), an SPR (Surface Plasmon Resonance), or an FET (Field Effect Transistor).

The "detection substance" may be a detection target substance which is subjected to detection in itself, or a substance which serves to indirectly detect a detection target substance. The detection substance is not limited to particular substances as long as it is a substance contained in a sample. Examples thereof include proteinaceous substances. In the case where the detection substance itself is a detection target substance, it is a disease-associated proteinaceous substance, for example. The proteinaceous substance is not limited to particular substances as long as it is an organic compound that contains amino acid. Examples thereof include protein, polypeptide, peptide, and modified products such as glycated products thereof. The disease-associated proteinaceous substance is not limited to particular substances as long as it is a proteinaceous substance that can be associated with a disease. Examples thereof include equol, hemoglobin F (HbF), HbA1c, and glycoalbumin. In the case where the detection substance is a substance which serves to indirectly detect a detection target substance, it is a ligand for a detection target substance, for example. Examples of the ligand include an antibody, an aptamer, and peptide.

The "correction substance" is not limited to particular substances as long as a substance that can be placed on the detecting section. Examples thereof may include an antibody, an aptamer, and peptide. Examples of the antibody may include a polyclonal antibody and a monoclonal antibody. Examples of the aptamer may include a RNA aptamer and a DNA aptamer.

On the detecting section, the reaction substance may be disposed in any position where a signal value derived from the reaction substance is detectable. For example, the reaction substance may be caused to bind to the upper surface of the detecting section.

The wording "the reaction substance can react specifically with each of the detection substance and the correction substance" means that the reaction substance selectively exerts an influence upon each of the detection substance and the correction substance. The reaction of the reaction substance may be any of a reversible reaction and an irreversible reaction as long as it is a reaction that can cause a change in the condition of the upper surface of the detecting section, and is not limited to particular reactions. Examples thereof may include a binding reaction, an enzymatic reaction, and a reduction reaction. Moreover, a substance other than the detection substance and the correction substance may exist as a substance with which the reaction substance specifically reacts. For example, a reaction between the correction substance and the reaction substance does not interfere with a reaction between the detection substance and the reaction substance.

For example, the "reaction substance" may be selected from the group consisting of compounds including protein, polypeptide, peptide, nucleic acid, and boronic acid, or may be a complex of a compound and a detection target substance, or a compound including a similar structure of a detection target substance. In the case where the detection substance itself is a detection target substance, the reaction substance may be a ligand. Examples of the ligand include an antibody, an aptamer, and peptide. Examples of the antibody may include a polyclonal antibody and a monoclonal antibody. Examples of the aptamer may include a RNA aptamer and a DNA aptamer. In the case where the detection substance is a substance which serves to indirectly detect a detection target substance, the reaction substance may be a substance labeled with a detection target substance. Examples thereof include a proteinaceous substance labeled with a detection target substance, and more specifically, 8-OHdG (8-hydroxy-2'-deoxyguanosine)-labeled BSA (bovine serum albumin) and PGDM (prostaglandin-D-metabolite)-labeled BSA. In the case where a plurality of substances of different types as exemplified herein are located on the detecting section, these substances may be collectively called "reaction substance".

In this specification, a reaction between the reaction substance and the detection substance is not limited to particular reactions as long as it is such a reaction that the reaction substance exerts an influence upon a sample containing the detection substance with consequent change in the condition of the upper surface of the detecting section. Examples of the reaction may include a binding reaction, an enzymatic reaction, and a reduction reaction.

The first supply step A21 is intended to supply the detection substance onto the detecting section.

The detection substance may be supplied in any given form onto the detecting section. For example, the detection substance is supplied, while being present as-is in a sample such as a biological sample, onto the detecting section. For example, the sample is diluted with a liquid such as a buffer solution prior to being supplied. Examples of the buffer solution include a phosphate buffer solution, a citrate buffer solution, a boric acid buffer solution, a HEPES (4- (2-hydroxyethyl)-1-piperidine ethane sulfonic acid) buffer solution, a tris (hydroxymethyl)aminomethane) buffer solution, and a MOPS (3-morpholinopropane sulfonic acid) buffer solution. The buffer solution is not limited to them, and may thus be any other known suitable buffer solution. The buffer solution may contain additional substances such as sodium chloride, potassium chloride, magnesium chloride, zinc chloride, and EDTA (ethylenediaminetetraacetate), and may further contain, on an as needed basis, a surfactant such as Tween 20 (registered trademark), Triton X-100 (registered trademark), and Brij 35 (registered trademark). Moreover, a blocking agent may be mixed in the buffer solution as required.

In this embodiment, the first measurement step A3 is intended to measure the first signal value based on a reaction between the detection substance and the reaction substance.

In this embodiment, the detecting section includes an elastic wave device, and, the first signal value may be a phase characteristic value of the elastic wave device. Moreover, in this embodiment, the first signal value may be a value measured by a quartz crystal microbalance (QCM) sensor, a surface plasmon resonance (SPR) sensor, or a field effect transistor (FET) sensor.

In this embodiment, the second supply step A22 is intended to supply the correction substance onto the detecting section.

The second supply step A22 may be carried out at any time under circumstances where the reaction substance undergoes reaction with the correction substance without interference so that the first signal value can be measured properly. For example, the second supply step A22 is carried out prior to the first supply step A21 or subsequent to the first measurement step A3.

In this embodiment, the correction substance may be supplied in any given form onto the detecting section. For example, the correction substance is diluted with a liquid such as a buffer solution prior to being supplied. For example, the correction substance is supplied in a known amount.

In this embodiment, the amount of the correction substance to be supplied in the second supply step A22 is not limited to particular values. For example, the correction substance is supplied in an excessive amount relative to the reaction substance.

In this embodiment, the second measurement step A4 is intended to measure the second signal value based on a reaction between the correction substance and the reaction substance.

In this embodiment, the detecting section may include an elastic wave device, and the second signal value may be a phase characteristic value of the elastic wave device. Moreover, in this embodiment, the second signal value may be a value measured by a quartz crystal microbalance sensor, a surface plasmon resonance sensor, or a field effect transistor sensor.

The second measurement step A4 may be carried out at any time under circumstances where the reaction substance undergoes reaction with the correction substance without interference so that the second signal value can be measured properly. For example, the second measurement step A4 is carried out prior to the first supply step A21 or subsequent to the first measurement step A3.

In this embodiment, the correction step A6 is intended to correct the first signal value with use of the second signal value.

In this embodiment, the correction step A6 may include calculating a sensitivity correction coefficient based on the second signal value, or based on the first signal value and the second signal value, and correcting the first signal value based on the calculated sensitivity correction coefficient. Moreover, in this embodiment, the first signal value may be corrected by an operation using the calculated sensitivity correction coefficient. For example, the first signal value may be divided by the second signal value.

The correction step A6 may be carried out at any time after the completion of the first measurement step A3 and the second measurement step A4.

As a modified example, a signal value amplifying step of amplifying the first signal value may be carried out between the first supply step A21 and the first measurement step A3. This permits amplification of the first signal value derived from the reaction substance, and thus enables the detection substance to be detected with higher sensitivity.

The first signal value can be amplified by reaction an amplification substance, which reacts specifically with the detection substance, with the detection substance.

For example, the "amplification substance" may be selected from the group consisting of compounds including protein, polypeptide, peptide, nucleic acid, and boronic acid. The amplification substance may be a ligand, for example. Examples of the ligand include an antibody, an aptamer, and peptide. Examples of the antibody may include a polyclonal antibody and a monoclonal antibody. Examples of the aptamer may include a RNA aptamer and a DNA aptamer.

A reaction between the amplification substance and the detection substance is not limited to particular reactions as long as it is such a reaction that the amplification substance exerts an influence upon a sample containing the detection substance with consequent change in the condition of the upper surface of the detecting section. Examples of the reaction may include a binding reaction, an enzymatic reaction, and a reduction reaction.

As a modified example, the amplification substance may be labeled with a labeling substance. This permits further amplification of the first signal value, and thus enables the detection substance to be detected with even higher sensitivity.

The "labeling substance" is not limited to particular substances as long as it serves to amplify at least the first signal value. Examples thereof may include biotin, enzyme, nanoparticles, and metallic nanoparticles.

Examples of the labeled amplification substance include a coenzyme labeled antibody such as a biotin labeled antibody, an enzyme labeled antibody such as a peroxidase labeled antibody and an alkaline phosphatase labeled antibody, a nanoparticle labeled antibody such as a metallic particle labeled antibody, enzyme labeled streptavidin such as peroxidase labeled streptavidin and alkaline phosphatase labeled streptavidin, and nanoparticle labeled streptavidin such as metallic particle labeled streptavidin.

The signal value amplifying step may be carried out at any time under circumstances where the reaction between the reaction substance and the detection substance, as well as the reaction between the reaction substance and the correction substance, proceeds without interference. For example, the signal value amplifying step may be carried out between the first supply step A21 and the first measurement step A3.

As a modified example, the measurement method further may include, subsequent to the first measurement step A3 and the second measurement step A4, a third supply step of supplying a correction substance which reacts specifically with the reaction substance onto the detecting section; and a third measurement step of measuring a third signal value based on a reaction between the correction substance and the reaction substance, and the first signal value can be corrected with use of the third signal value in the correction step A6. This permits correction for variations between the detecting sections after the amplification of the first signal value, and thus enables the detection substance to be measured with greater precision and with higher sensitivity.

The third supply step may be carried out at any time under circumstances where the reaction substance undergoes reaction with the detection substance without interference so that the first signal value can be measured properly. For example, the third supply step may be carried out subsequent to the first measurement step A3 and the second measurement step A4.

In this case, the correction substance may be supplied in any given form onto the detecting section. For example, the correction substance is diluted with a liquid such as a buffer solution prior to being supplied.

Moreover, the detecting section includes an elastic wave device, and the third signal value may be a phase characteristic value of the elastic wave device. Furthermore, in this embodiment, the third signal value may be a value measured by a quartz crystal microbalance sensor, a surface plasmon resonance sensor, or a field effect transistor sensor.

Moreover, the correction step A6 may include calculating a sensitivity correction coefficient based on the third signal value, or based on the first signal value and the third signal value, and correcting the first signal value based on the calculated sensitivity correction coefficient. In this embodiment, alternatively, the first signal value may be corrected by an operation using the calculated sensitivity correction coefficient. For example, the first signal value may be divided by the calculated sensitivity correction coefficient.

As a modified example, a removal step of removing the correction substance from the detecting section may be additionally carried out subsequent to the second measurement step A4. This eliminates the influence of the second signal value derived from the correction substance, and thus permits more accurate measurement of the first signal value derived from the reaction substance. Consequently, the detection substance can be measured more accurately.

At a point of time when the second measurement step A4 was completed, the correction substance had already reacted with the reaction substance, and the measurement of the second signal value derived from the correction substance had already come to an end. Thus, after the second measurement step A4, the removal of the correction substance from on the detecting section does not affect the second signal value. Accordingly, in the case where the first measurement step A3 is carried out subsequent to the second measurement step A4, with the removal of the correction substance after reaction with the reaction substance from on the detecting section, the influence of the correction substance can be eliminated. This allows the first signal value based on the reaction between the detection substance and the reaction substance to be exclusively detected with accuracy.

As a modified example, a blocking step of carrying out blocking treatment on the detecting section with a blocking agent which is non-specifically bindable to the detecting section may be carried out subsequent to the preparation step A1 and prior to the first supply step A21 and the second supply step A22. This enables the detection substance, the correction substance, the second correction substance, and the amplification substance to exert an influence upon the detecting section without undergoing reaction with the reaction substance, and thus can prevent occurrence of errors in the first signal value, the second signal value, and the third signal value.

The "blocking agent" is not limited to particular substances as long as it is a substance that differs from each of the detection substance, the reaction substance, the correction substance, and the amplification substance. Examples of the blocking agent include BSA, casein, polyethylene glycol, a MPC (2-methacryloyloxythyl phosphorylcholine) polymer, a betaine polymer, and a HEMA (hydroxyethyl methacrylate) polymer.

The wording "carry out blocking treatment on the detecting section" means that the blocking agent covers the detecting section so that other substances do not approach the detecting section.

The blocking step may be carried out at any time under circumstances where the reaction between the reaction substance and the detection substance, as well as the reaction between the reaction substance and the correction substance, proceeds without interference. For example, the blocking step may be carried out subsequent to the preparation step A1 and prior to the first supply step A21 and the second supply step A22. In the case of carrying out the blocking step prior to the first supply step A21, it is possible to increase the accuracy of the first signal value. In the case of carrying out the blocking step between the first supply step A21 and the second supply step A22, it is possible to increase the accuracy of the second signal value.

As a modified example, a first washing step of supplying a washing solution onto the detecting section may be carried out between the first supply step A21 and the first measurement step A3. This permits the removal of, for example, foreign matter present in the sample containing the detection substance, from on the detecting section, and thus can eliminate errors resulting from the foreign matter, etc. Consequently, the detection substance can be measured more accurately.

In this specification, the "washing solution" is not limited to particular solutions as long as it does not exert any influence upon the first signal value, the second signal value, and the third signal value. Examples thereof may include a buffer solution.

As a modified example, a second washing step of supplying a washing solution onto the detecting section may be carried out between the first supply step A21 and the signal value amplifying step. This permits the removal of amplification substances that are not reactive with the detection substance from on the detecting section, and thus can eliminate errors resulting from unreacted amplification substances. Consequently, the detection substance can be accurately measured with high sensitivity.

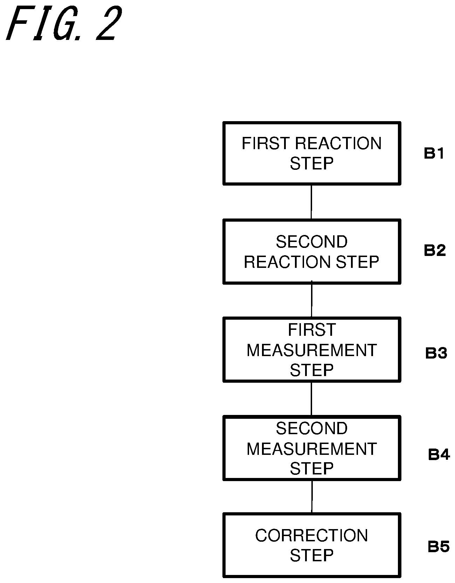

FIG. 2 is a flow chart for a modified example of the measurement method according to one embodiment.

The measurement method according to the invention includes:

a first reaction step B1 of reacting a detection substance with a reaction substance which reacts specifically with each of the detection substance and a correction substance;
a second reaction step B2 of reacting the correction substance with the reaction substance;
a first measurement step B3 of measuring a first signal value derived from a reaction between the detection substance and the reaction substance;
a second measurement step B4 of measuring a second signal value derived from a reaction between the correction substance and the reaction substance; and
a correction step B5 of correcting the first signal value with use of the second signal value, and
the second reaction step B2 and the second measurement step B4 may be carried out prior to the first reaction step B1 or subsequent to the first measurement step B3.

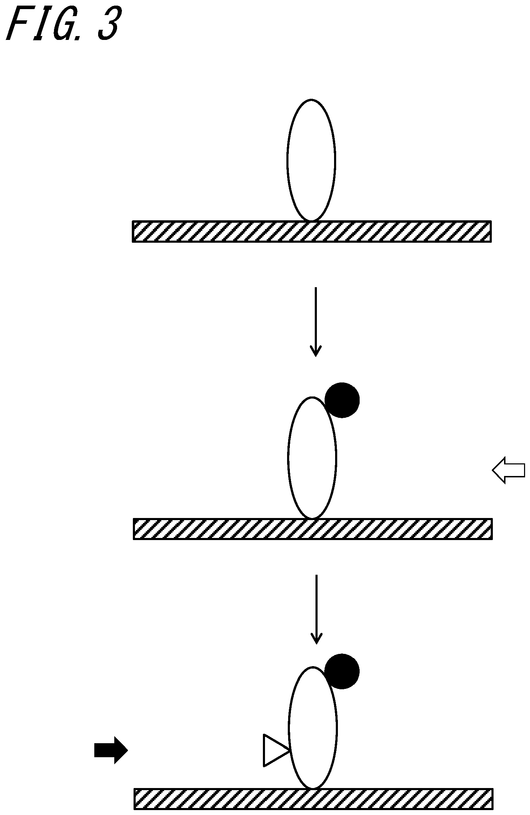

FIG. 3 is a diagram schematically showing, as an example of one embodiment, a direct assay in which the detection substance itself is detected as a detection target substance.

In this example, the detection substance is hemoglobin; an elastic wave device is used as the detecting section; an anti-hemoglobin antibody is used as the reaction substance; and a secondary antibody against the anti-hemoglobin antibody is used as the correction substance.

In FIG. 3, the diagonally shaded rectangle represents the elastic wave device; the open ellipse represents an anti-hemoglobin antibody serving as the reaction substance; the solid filled circle represents hemoglobin serving as the detection substance; and the open triangle represents a secondary antibody serving as the correction substance. Moreover, the open arrow represents the point of time at which the first signal value is measured, and the solid filled arrow represents the point of time at which the second signal value is measured.

That is, an example of one embodiment includes:

a preparation step A1 of preparing an elastic wave device on au upper surface of which an anti-hemoglobin antibody that can react with hemoglobin and a secondary antibody is placed and which can measure a signal value based on a reaction of the anti-hemoglobin antibody;
a first supply step A21 of supplying the hemoglobin onto the elastic wave device;
a second supply step A22 of supplying the secondary antibody onto the elastic wave device;
a first measurement step A3 of measuring a first signal value based on a specific reaction between the hemoglobin and the anti-hemoglobin antibody;
a second measurement step A4 of measuring a second signal value based on a specific reaction between the secondary antibody and the anti-hemoglobin antibody; and
a correction step A5 of correcting the first signal value with use of the second signal value.

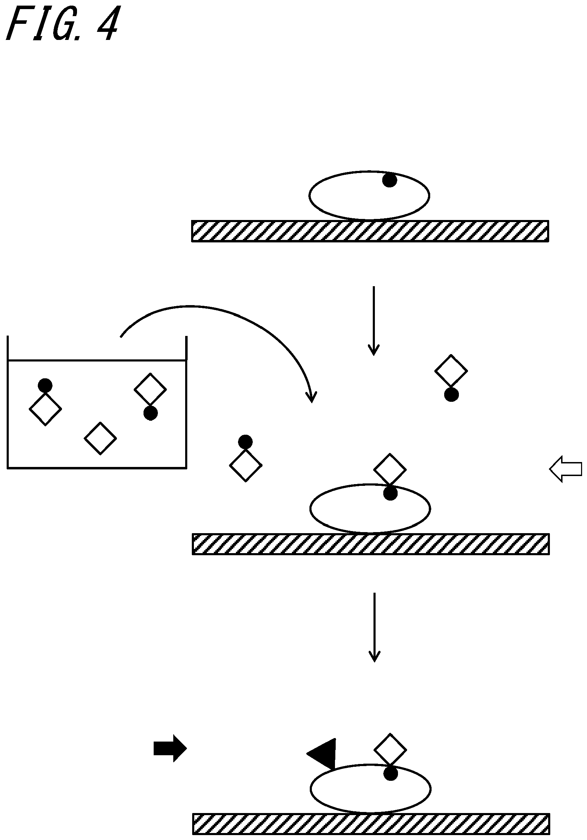

FIG. 4 is a diagram schematically showing, as an example of one embodiment, an indirect assay in which the detection substance is detected to indirectly detect a detection target substance.

In this example, the detection target substance is 8-OHdG; the detection substance is an anti-8-OHdG antibody; an elastic wave device is used as the detecting section; labeled BSA is used as the reaction substance; and an anti-BSA antibody is used as the correction substance.

In FIG. 4, the diagonally shaded rectangle represents the elastic wave device; the open ellipse represents BSA serving as the reaction substance; the solid filled circle represents 8-OHdG which is the detection target substance; the open rectangle represents an anti-8-OHdG antibody serving as the detection substance; and the solid filled triangle represents an anti-BSA antibody serving as the correction substance. Note that the solid filled circle within the open ellipse represents 8-OHdG for labeling BSA. Moreover, the open arrow represents the point of time at which the first signal value is measured, and the solid filled arrow represents the point of time at which the second signal value is measured.

That is, an example of one embodiment includes:

a preparation step A1 of preparing an elastic wave device on an upper surface of which BSA that can react with an anti-8-OHdG antibody and an anti-BSA antibody is placed and which can measure a signal value based on a reaction of the BSA;
a first supply step A21 of supplying an anti-8-OHdG antibody onto the elastic wave device;
a second supply step A22 of supplying an anti-BSA antibody onto the elastic wave device;
a first measurement step A3 of measuring a first signal value based on a specific reaction between the anti-8-OHdG antibody and the BSA;
a second measurement step A4 of measuring a second signal value based on a specific reaction between the anti-BSA antibody and the BSA; and
a correction step A5 of correcting the first signal value with use of the second signal value.

(Another embodiment)

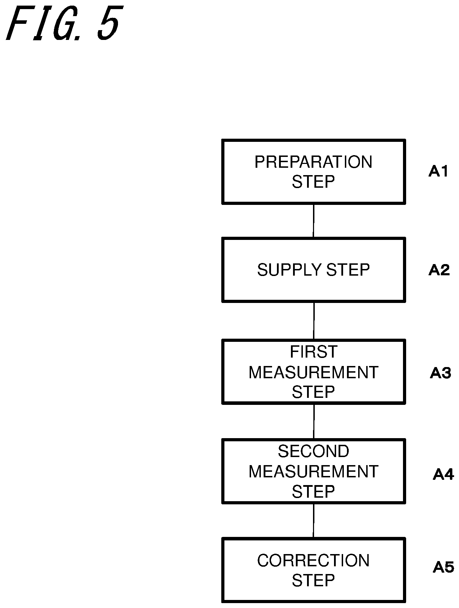

FIG. 5 is a flow chart for the measurement method according to another embodiment of the invention. In FIG. 5, the first supply step A21 and the second supply step A22 are collectively shown as "supply step A2".

In the measurement method according to another embodiment,
the detecting section includes: a first detecting section on an upper surface of which a first reaction substance which can react specifically with a detection substance is placed, and a second detecting section on an upper surface of which a second reaction substance which can react specifically with a correction substance is placed, and
the correction substance specifically can react at least with the second reaction substance, and the detection substance and the correction substance are supplied in an order or simultaneously.

In the measurement method according to another embodiment, the second reaction substance is not limited to particular substances as long as it is a substance that can be placed on the second detecting section and is not specifically reactive with the detection substance. In the measurement method according to another embodiment, examples of the second reaction substance may include BSA (bovine serum albumin), protein such as casein, a polymer, nucleic acid, an antibody, an aptamer, and peptide. Examples of the antibody may include a polyclonal antibody and a monoclonal antibody. Examples of the aptamer may include a RNA aptamer and a DNA aptamer.

With respect to the term "placed on an upper surface" in the description regarding the first detecting section, the first reaction substance may be disposed in any position on the first detecting section where it serves to detect a signal value derived from the first reaction substance. For example, the first reaction substance may be caused to bind to the first detecting section. Moreover, after binding to the surface of the first detecting section, the first reaction substance may be separated from the surface.

With respect to the term "placed on an upper surface" in the description regarding the second detecting section, the second reaction substance may be disposed in any position on the second detecting section where it serves to detect a signal value derived from the second reaction substance. For example, the second reaction substance may be caused to bind to the second detecting section. Moreover, after binding to the surface of the second detecting section, the second reaction substance may be separated from the surface.

The term "the first reaction substance which can react specifically with the detection substance" means that the first reaction substance and the detection substance selectively react with each other. The reaction of the first reaction substance may be any of a reversible reaction and an irreversible reaction as long as it is a reaction that can cause a change in the condition of the upper surface of the first detecting section, but is not limited to particular reactions. Examples thereof may include a binding reaction, an enzymatic reaction, an antigen-antibody reaction, and a reduction reaction. While the first reaction substance may react specifically with a substance other than the detection substance, the first reaction substance preferably reacts specifically only with the detection substance.

In the measurement method according to another embodiment, for example, the "first reaction substance" may be selected from the group consisting of compounds including protein, polypeptide, peptide, nucleic acid, and boronic acid, or may be a complex of a compound and the detection substance, or a compound including a similar structure of the detection substance. For example, the first reaction substance may be a ligand. Examples of the ligand include an antibody, an aptamer, and peptide. Examples of the antibody may include a polyclonal antibody and a monoclonal antibody. Examples of the aptamer may include a RNA aptamer and a DNA aptamer.

In the measurement method according to another embodiment, the supply step A2 is intended to supply the correction substance, which specifically reacts at least with the second reaction substance, and the detection substance in an order or simultaneously onto the first detecting section, as well as onto the second detecting section.

The term "the correction substance which specifically reacts at least with the second reaction substance" means that the correction substance and the second reaction substance selectively react with each other. The correction substance may react selectively with a substance other than the second reaction substance. The substance other than the second reaction substance may be the detection substance, for example. The reaction between the correction substance and the second reaction substance may be any of a reversible reaction and an irreversible reaction as long as it is a reaction that can cause a change in the condition of the upper surface of the second detecting section, but is not limited to particular reactions. Examples thereof may include a binding reaction, an enzymatic reaction, an antigen-antibody reaction, and a reduction reaction.

For example, a reaction between the correction substance and the substance other than the second reaction substance does not interfere with a reaction between the correction substance and the second reaction substance. This makes it possible to simultaneously supply the correction substance and the detection substance onto the first and second detecting sections, and thus to shorten the measurement time.

In this specification, the term "simultaneously supply the detection substance and the correction substance" means that the detection substance and the correction substance are supplied together, and more specifically, the term is applied not only to the case of supplying the detection substance and the correction substance at exactly the same time, but also to the case of supplying the detection substance and the correction substance at slightly different times. For example, in the case where the detection substance and the correction substance are present in one and the same liquid, the detection substance and the correction substance may reach the first detecting section or the second detecting section at different times during the supply of the liquid.

In this embodiment, the detection substance and the correction substance may be supplied in an order onto the first and second detecting sections. The term "in an order" is used merely to mark a distinction from the term "simultaneously" as above described, and hence any way of supply other than the simultaneous supply may be adopted. As an example, after the detection substance is supplied onto the first and second detecting sections, the correction substance is supplied onto the first and second detecting sections, and vice versa, that is; after the correction substance is supplied onto the first and second detecting sections, the detection substance is supplied onto the first and second detecting sections. As another example, the supply of the correction substance is started in the course of supplying the detection substance.

In this embodiment, a washing step may be carried out between the supply of the correction substance and the supply of the detection substance. This enables measurement to be effected with greater precision than would be the case with the simultaneous supply of the detection substance and the correction substance. For example, in the case where the detection substance and the correction substance are present in different liquids, the liquid containing the detection substance is first supplied; the surfaces of the first and second detecting sections are then washed with a washing solution; and thereafter the liquid containing the correction substance is supplied.

The wording "supplied onto the first and second detecting sections" is applied not only to a case where the detection substance and the correction substance are simultaneously supplied onto the first and second detecting sections at one time, but also to cases where, after one of the detection substance and the correction substance is supplied onto the first detecting section, the other is supplied onto the second detecting section, and after one of the detection substance and the correction substance is supplied onto the second detecting section, the other is supplied onto the first detecting section. For example, a liquid containing the detection substance and a liquid containing the correction substance may be supplied so that they can contact the surfaces of the first and second detecting sections at exactly the same time or at slightly different times, or alternatively, the liquids may be supplied so that they cover the surface of the first detecting section completely, and then cover the surface of the second detecting section.

In this embodiment, the detection substance and the correction substance may be supplied to the first detecting section and the second detecting section simultaneously, or may be supplied to the first detecting section first, and be then supplied to the second detecting section. The correction substance may be supplied together with the detection substance to the first detecting section and the second detecting section simultaneously. The detection substance may be supplied prior to or subsequent to the supply of the correction substance. For example, the correction substance may be supplied alone to the first detecting section first, and be then supplied to the second detecting section. After that, the detection substance may be supplied alone to the first detecting section first, and be then supplied to the second detecting section.

For example, the detection substance may be supplied simultaneously with the correction substance onto the first detecting section, and be then supplied simultaneously with the correction substance onto the second detecting section. This enables measurement to be accomplished in a shorter period of time than would be the case where the detection substance and the correction substance are supplied in an order, and also enables measurement to be carried out with greater precision and higher sensitivity than would be the case where the first detecting section and the second detecting section are supplied with the substances at the same time. That is, excellent balance can be achieved between measurement time and measurement precision and measurement sensitivity.

In this embodiment, for example, the "correction substance" may be selected from the group consisting of compounds including protein, polypeptide, peptide, nucleic acid, and boronic acid. For example, the correction substance may be a ligand. Examples of the ligand include an antibody, an aptamer, and peptide. Examples of the antibody may include a polyclonal antibody and a monoclonal antibody. Examples of the aptamer may include a RNA aptamer and a DNA aptamer.

In this embodiment, the correction substance may be supplied in any given form onto the first and second detecting sections. For example, the correction substance is diluted with a liquid such as a buffer solution prior to being supplied onto the first and second detecting sections.

In this embodiment, the detection substance may be supplied in any given form. For example, the detection substance is supplied while being present as-is in a sample such as a biological sample. For example, the sample is diluted with a liquid such as a buffer solution prior to being supplied. Moreover, the detection substance may be blended with the correction substance in a buffer solution prior to being supplied.

In this embodiment, examples of the buffer solution include a phosphate buffer solution, a citrate buffer solution, a boric acid buffer solution, a HEPES (4- (2-hydroxyethyl)-1-piperidine ethane sulfonic acid) buffer solution, a Tris (tris (hydroxymethyl) aminomethane) buffer solution, and a MOPS (3-morpholinopropane sulfonic acid) buffer solution. The buffer solution is not limited to them, and may thus be any other known suitable buffer solution. The buffer solution may contain additional substances such as sodium chloride, potassium chloride, magnesium chloride, zinc chloride, and EDTA (ethylenediaminetetraacetate), and may further contain, on an as needed basis, a surfactant such as Tween 20 (registered trademark), Triton X-100 (registered trademark), and Brij 35 (registered trademark). Moreover, a blocking agent may be mixed in the buffer solution as required.

In this embodiment, the "first signal value" may be a signal value based on a change in the condition of the upper surface of the first detecting section resulting from a specific reaction between the detection substance and the first reaction substance.

In this embodiment, the first detecting section may include an elastic wave device, and the first signal value may be a phase characteristic value of the elastic wave device. Moreover, in this embodiment, the first signal value may be a value measured by a QCM sensor, an SPR sensor, or an FET sensor.

In this embodiment, the "second signal value" may be a signal value based on a change in the condition of the upper surface of the second detecting section resulting from a specific reaction between the second reaction substance and the correction substance.

In this embodiment, the second detecting section may include an elastic wave device, and the second signal value may be a phase characteristic value of the elastic wave device. Moreover, in this embodiment, the second signal value may be a value measured by a QCM sensor, an SPR sensor, or an FET sensor.

For example, the second signal value is measured after the reaction between the second reaction substance and the correction substance reaches a level of saturation. This makes it possible to adjust the amount of the correction substance which reacts with a measured amount of the second reaction substance to a predetermined amount, and thereby measure the second signal value with high precision.

In this embodiment, the second measurement step A4 may be carried out after implementation of the first measurement step A3, or alternatively, the first measurement step A3 may be carried out after implementation of the second measurement step A4.

In this embodiment, the correction step A5 is intended to correct the first signal value with use of the second signal value. Correcting the first signal value with the second signal value allows correction for variations in sensitivity between individual detecting elements.

The first detecting sections provided in different detecting elements differ from each other in upper surface condition, which results in variations in sensitivity between the detecting elements. In the case where the first detecting section and the second detecting section are located on one and the same detecting element, there is no difference in upper surface condition between them. The second detecting section for correction is located on one detecting element on which the first detecting section for substantially measuring values of a detection target substance is located, and hence the first detecting section and the second detecting section exhibit the same sensitivity. Thus, the first signal value derived from the upper surface condition of the first detecting section can be corrected with use of the second signal value derived from the upper surface condition of the second detecting section. This permits highly precise measurements on substances contained in a sample.

In this embodiment, the correction step A5 may include calculating a sensitivity correction coefficient based on the second signal value, or based on the first signal value and the second signal value, and correcting the first signal value based on the calculated sensitivity correction coefficient. Moreover, in this embodiment, the first signal value is preferably corrected by an operation using the calculated sensitivity correction coefficient.

In this embodiment, for example, the first signal value may be divided by the second signal value. The operation on the first signal value may be carried out in accordance with the following formulae for elimination of the influence of element sensitivity:

\<First Detecting Element\>

$$\text{First signal value} \propto \text{element sensitivity A} \times$$

$$\text{correction substance binding amount } \alpha \quad \text{(Formula 1)};$$

$$\text{Second signal value} \propto \text{element sensitivity A} \times$$

$$\text{correction substance binding amount } \beta \quad \text{(Formula 2)};$$

<Second Detecting Element>

$$\text{First signal value} \propto \text{element sensitivity B} \times$$

$$\text{correction substance binding amount } \alpha \quad \text{(Formula 3)};$$

and

$$\text{Second signal value} \propto \text{element sensitivity B} \times$$

$$\text{correction substance binding amount } \beta \quad \text{(Formula 4)}.$$

In each of the first detecting element and the second detecting element, by dividing the first signal value by the second signal value, the following Formula 5 holds:

$$\text{First signal value/second signal value} \propto \text{correction}$$

$$\text{substance binding amount } \alpha/\text{correction substance binding}$$

$$\text{amount } \beta \quad \text{(Formula 5)}.$$

Thus, as seen from Formula 3, the value of [correction substance binding amount $\alpha$/correction substance binding amount $\beta$] can be calculated without the influence of the element sensitivity A. Note that the correction substance binding amount $\alpha$ represents the amount of the correction substance bound to the first detecting section, and the correction substance binding amount $\beta$ represents the amount of the correction substance bound to the second detecting section. Calculation of the value of [$\alpha/\beta$] permits the elimination of the influence of element sensitivity, and thus allows correction for variations in sensitivity between the first detecting sections. The first detecting section and the second detecting section in the first detecting element exhibit the same element sensitivity A, and, the first detecting section and the second detecting section in the second detecting element exhibit the same element sensitivity B. The correction substance is supplied in a known amount relative to the second reaction substance.

As a modified example, in the first supply step A2, the detection substance and the correction substance may be supplied in an order or simultaneously to a part of the surface of a third detecting section which is free of the first reaction substance and the second reaction substance; a third measurement step of measuring a third signal value based on the upper surface condition of the third detecting section may be additionally carried out; and in the correction step A5, the first signal value may be corrected with use of the third signal value.

For example, the "third detecting section" is provided at its upper surface with a detecting element for producing signal value output. Examples of the detecting element may include, but is not limited to, an elastic wave device, a QCM, an SPR, or an FET.

In this embodiment, the "third signal value" refers to a signal value derived from the condition of the upper surface of the third detecting section that is insusceptible to any reaction derived from the detection substance and the correction substance. The third signal value is thus used as a control value.

The third detecting section, which bears no first and second reaction substances on an upper surface thereof, is used to acquire a control value. The surface of the third detecting section may be subjected to blocking treatment using, for example, a blocking agent. This can eliminate the influence of foreign matter, and thus enables substances contained in a sample to be detected with even higher precision.

For example, the detection substance is supplied simultaneously with the correction substance onto the third detecting section, is then supplied simultaneously with the correction substance onto the first detecting section, and is lastly supplied together with the correction substance onto the second detecting section. In this case, excellent balance can be achieved among measurement time and measurement precision and measurement sensitivity.

As a modified example, a signal value amplifying step of amplifying a signal value based on a reaction between the first reaction substance and the detection substance may be carried out between the first supply step A2 and the first measurement step A3. This enables the detection substance to be detected with higher sensitivity.

In the signal value amplifying step, the signal value based on the reaction between the first reaction substance and the detection substance may be amplified by reaction an amplification substance, which reacts specifically with the detection substance, with the detection substance.

In this embodiment, as the "amplification substance", an amplification substance of the same kind as that exemplified in the preceding embodiment may be used.

A reaction between the third reaction substance and the detection substance is not limited to particular reactions as long as it is such a reaction that the third reaction substance exerts an influence directly or indirectly upon the detection substance with consequent change in the condition of the upper surface of the first detecting section. Examples of the reaction may include a binding reaction, an enzymatic reaction, an antigen-antibody reaction, and a reduction reaction.

In this embodiment, the signal value amplifying step may be carried out at any time under circumstances where the reaction between the first reaction substance and the detection substance, as well as the reaction between the correction substance and the second reaction substance, proceeds without interference. For example, the signal value amplifying step may be carried out between the first supply step A2 and the first measurement step A3.

In the correction step A5 in this embodiment, for example, the third signal value may be subtracted from the first signal value, as well as from the second signal value. This can eliminate a signal derived from non-specific binding of the detection substance, the correction substance, and other substances to the surfaces of the first and second detecting sections, which could be contained in the first signal value and in the second signal value. Consequently, measurements can be carried out with even higher precision.

As a modified example, in the measurement method according to another embodiment, a blocking step of carrying out blocking treatment on the surfaces of the first and second detecting sections with the supply of a blocking agent which is non-specifically bindable to the surfaces of the first and second detecting sections may be carried out. This can protect the first detecting section, the second detecting section, and the third detecting section from the influence of foreign matter, and thus can prevent occurrence of errors in the first signal value, the second signal value, and the third signal value more reliably.

In this embodiment, the "blocking agent" may be any substance that differs from each of the detection substance, the first reaction substance, the second reaction substance, the correction substance, and the amplification substance. Thus, a blocking agent of the same kind as that exemplified in the preceding embodiment may be used.

The wording "carrying out blocking treatment" means that the blocking agent covers the surfaces of the first, second, and third detecting sections to avoid non-specific adsorption of other substances to the surfaces of the first, second, and third detecting sections.

The blocking step may be carried out at any time under circumstances where the reaction between the first reaction substance and the detection substance, as well as the reaction between the second reaction substance and the correction substance, proceeds without interference. For example, the blocking step may be carried out subsequent to the preparation step A1 and prior to the first supply step A2. By carrying out the blocking step prior to the first supply step A2, it is possible to eliminate the influence of foreign matter, and thus to increase the accuracy of the first signal value.

As a modified example, a washing step of supplying a washing solution onto the first, second, and third detecting sections may be carried out. This permits the removal of foreign matter present in the sample containing the detection substance from on the first, second, and third detecting sections, and thus can eliminate errors resulting from the foreign matter. Consequently, the detection substance can be measured more accurately.

In this specification, the "washing solution" is not limited to particular solutions as long as it does not exert any influence upon the first signal value, the second signal value, and the third signal value. Examples thereof may include a buffer solution.

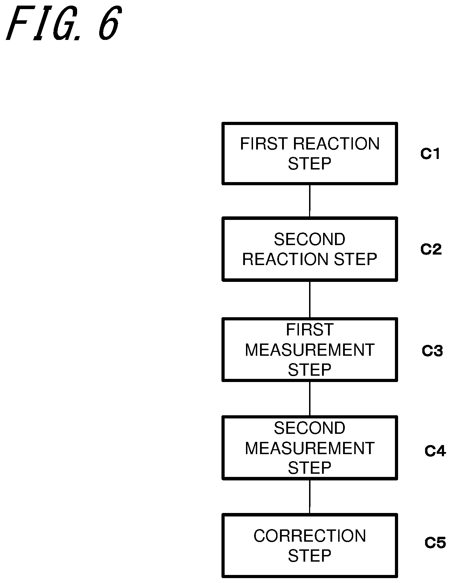

FIG. 6 is a flow chart for a modified example of the measurement method according to another embodiment.

A modified example of the measurement method according to the embodiment includes:

a first reaction step C1 of reacting a detection substance with a first reaction substance which reacts specifically with the detection substance;

a second reaction step C2 of reacting a second reaction substance with a correction substance which specifically reacts at least with the second reaction substance;

a first measurement step C3 of measuring a first signal value derived from a reaction between the detection substance and the first reaction substance;

a second measurement step C4 of measuring a second signal value derived from a reaction between the second reaction substance and the correction substance; and

a correction step C5 of correcting the first signal value with use of the second signal value.

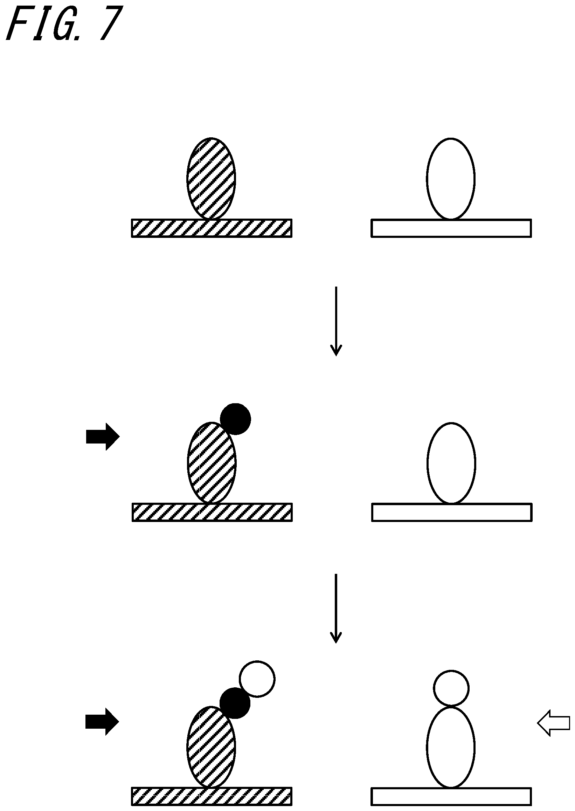

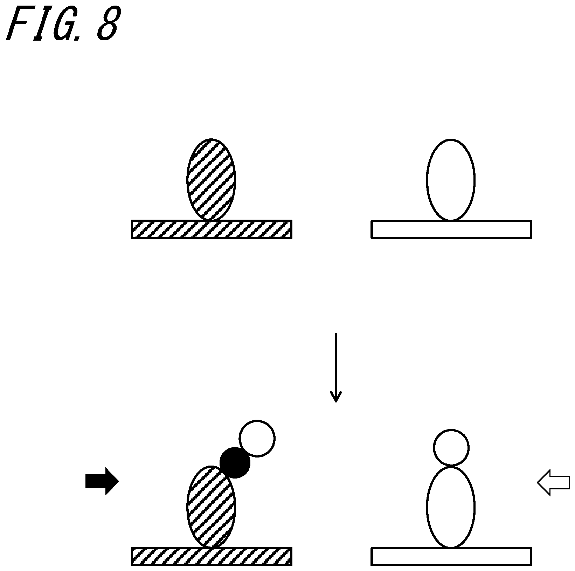

FIGS. 7 and 8 are diagrams each schematically showing, as an example of another embodiment, an assay in which the correction substance reacts specifically with the detection substance and the second reaction substance. FIG. 3 shows a case where the correction substance is supplied after the supply of the detection substance. FIG. 4 shows a case where the correction substance and the detection substance are supplied simultaneously.

In FIGS. 7 and 8, the diagonally shaded open rectangle represents the first detecting section; the open rectangle represents the second detecting section; the diagonally shaded open ellipse represents the first reaction substance; the open ellipse represents the second reaction substance; the solid filled circle represents the detection substance; and the open circle represents the correction substance. Moreover, the solid filled arrow represents the point of time at which the first signal value is acquired, and the open arrow represents the point of time at which the second signal value is acquired.

In this example, the detection substance is hemoglobin; an elastic wave device is used as each of the first detecting section and the second detecting section; a first anti-hemoglobin antibody, which will hereafter be referred to as "anti-hemoglobin antibody", is used as the first reaction substance; a second anti-hemoglobin antibody, which will hereafter be referred to as "antibody for sandwich assay", is used as the correction substance; and a secondary antibody is used as the second reaction substance. The first anti-hemoglobin antibody and the second anti-hemoglobin antibody recognize different epitopes.

That is, an example of another embodiment includes:

a preparation step A1 of preparing a detecting section on an upper surface of which a reaction substance (an anti-hemoglobin antibody and a secondary antibody) that can react with hemoglobin and an antibody for sandwich assay is placed and which can measure a signal value based on a reaction of the reaction substance;

a supply step A2 of supplying hemoglobin and an antibody for sandwich assay onto an elastic wave device (a first elastic wave device and a second elastic wave device);

a first measurement step A3 of measuring a first signal value based on a specific reaction between the hemoglobin and the anti-hemoglobin antibody;

a second measurement step A4 of measuring a second signal value based on a specific reaction between the antibody for sandwich assay and the secondary antibody; and

a correction step A5 of correcting the first signal value with use of the second signal value.

In the above-described example, the antibody for sandwich assay specifically can react at least with the secondary antibody, and the hemoglobin and the antibody for sandwich assay are supplied in an order or simultaneously onto the first elastic wave device and the second elastic wave device.

The first signal value may be a signal value based on a reaction between the hemoglobin and the antibody for sandwich assay. In this case, the signal value can be measured with higher sensitivity than would be the case with a signal value based on a reaction between the hemoglobin and the first reaction substance.

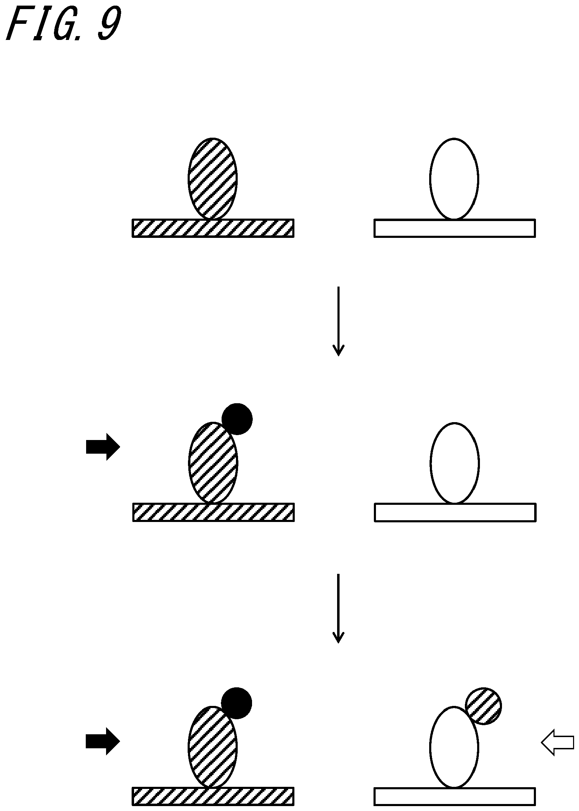

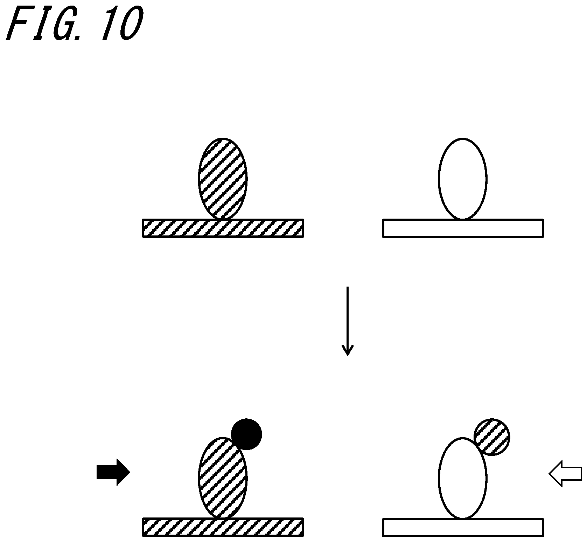

FIGS. 9 and 10 are diagrams each schematically showing, as an example of another embodiment, an assay in which the correction substance reacts specifically with the second reaction substance only. FIG. 9 shows a case where the correction substance is supplied after the supply of the detection substance. FIG. 10 shows a case where the correction substance and the detection substance are supplied simultaneously.

In FIGS. 9 and 10, the diagonally shaded open rectangle represents the first detecting section; the open rectangle represents the second detecting section; the diagonally shaded open ellipse represents the first reaction substance; the open ellipse represents the second reaction substance; the solid filled circle represents the detection substance; and the diagonally shaded open circle represents the correction substance. Moreover, the solid filled arrow represents the point of time at which the first signal value is acquired, and the open arrow represents the point of time at which the second signal value is acquired.

In this example, the detection substance is hemoglobin; an elastic wave device is used as each of the first detecting section and the second detecting section; an anti-hemoglobin antibody is used as the first reaction substance; BSA is used as the correction substance; and an anti-BSA antibody is used as the second reaction substance.

That is, an example of another embodiment includes:

a preparation step A1 of preparing an elastic wave device (a first elastic wave device and a second elastic wave device) on an upper surface of which a reaction substance (an anti-hemoglobin antibody and an anti-BSA antibody) that can react with hemoglobin and BSA is placed and which can measure a signal value based on a reaction of the reaction substance;
a supply step A2 of supplying hemoglobin and BSA onto the elastic wave device;
a first measurement step A3 of measuring a first signal value based on a specific reaction between the hemoglobin and the anti-hemoglobin antibody;
a second measurement step A4 of measuring a second signal value based on a specific reaction between the BSA and the anti-BSA antibody; and
a correction step A5 of correcting the first signal value with use of the second signal value.

In the above-described example, the BSA specifically reacts at least with the anti-BSA antibody, and the hemoglobin and the BSA are supplied in an order or simultaneously onto the first elastic wave device and the second elastic wave device.

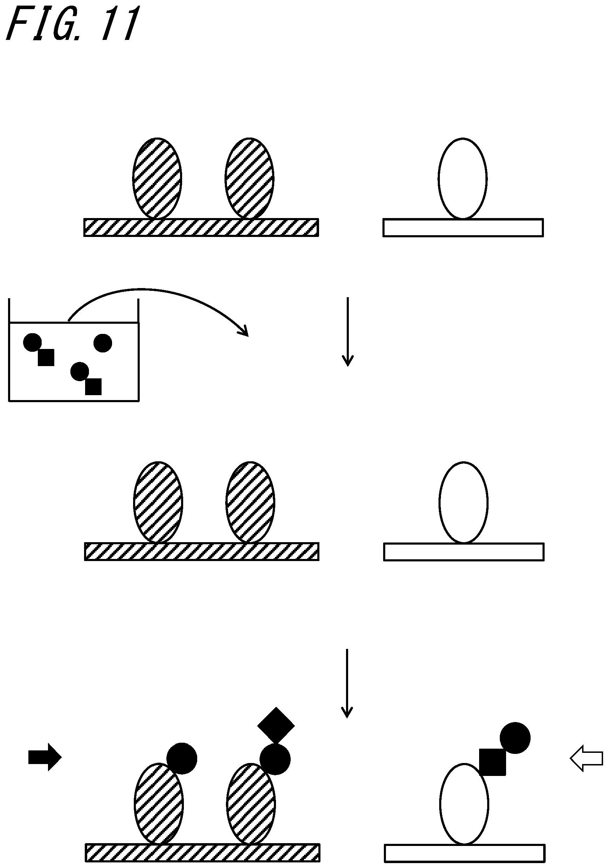

FIG. 11 is a diagram schematically showing, as an example of another embodiment, an assay in which a mixture of the detection substance and the correction substance is supplied.

In FIG. 11, the diagonally shaded open rectangle represents the first detecting section; the open rectangle represents the second detecting section; the diagonally shaded open ellipse represents the first reaction substance; the open ellipse represents the second reaction substance; the solid filled circle represents the detection substance; and the combination of solid filled square and solid filled circle represents the correction substance. Moreover, the solid filled arrow represents the point of time at which the first signal value is acquired, and the open arrow represents the point of time at which the second signal value is acquired.

In this example, the detection substance is 8-OHdG; an elastic wave device is used as the first detecting section and the second detecting section; an anti-8-OHdG antibody is used as the first reaction substance; 8-OHdG-labeled BSA is used as the correction substance; and an anti-BSA antibody against the labeling substance is used as the second reaction substance.

That is, an example of another embodiment includes:

a preparation step A1 of preparing an elastic wave device (a first elastic wave device and a second elastic wave device) on an upper surface of which a reaction substance (an anti-8-OHdG antibody and an anti-BSA antibody against the labeling substance) that can react with 8-OHdG and 8-OHdG-labeled BSA is placed and which can measure a signal value based on a reaction of the reaction substance;
a supply step A2 of supplying 8-OHdG and 8-OHdG-labeled BSA onto the elastic wave device;
a first measurement step A3 of measuring a first signal value based on a specific reaction between the 8-OHdG and the anti-8-OHdG antibody;
a second measurement step A4 of measuring a second signal value based on a specific reaction between the 8-OHdG-labeled BSA and the anti-BSA antibody against the labeling substance; and
a correction step A5 of correcting the first signal value with use of the second signal value.

In the above-described example, the 8-OHdG-labeled BSA specifically reacts at least with the anti-BSA antibody against the labeling substance, and the 8-OHdG and the 8-OHdG-labeled BSA are supplied in an order or simultaneously onto the first elastic wave device and the second elastic wave device.

<Measurement Device>

(One Embodiment)

The following describes one embodiment of the invention with respect to a case where the detection substance is hemoglobin. Note that the following example is given merely as one embodiment of the invention, and the scope of the invention is not limited to the embodiment as set forth hereinafter.

A measurement device 100 according to the embodiment includes:

a detecting section on an upper surface of which a reaction substance that can react with a detection substance and a correction substance is placed and which can measure a signal value based on a reaction of the reaction substance;
a supply section which supplies the detection substance and the correction substance to the detecting section;

a supply section which supplies the detection substance and the correction substance onto the detecting section;
a measurement section which measures a first signal value based on a reaction between the detection substance and the reaction substance, and a second signal value based on a reaction between the correction substance and the reaction substance; and
a correction section which corrects the first signal value with use of the second signal value.

In the measurement device according to the above-described embodiment,
the reaction substance can react specifically with each of the detection substance and the correction substance, and the measurement section measures the second signal value prior to or subsequent to the measurement of the first signal value.

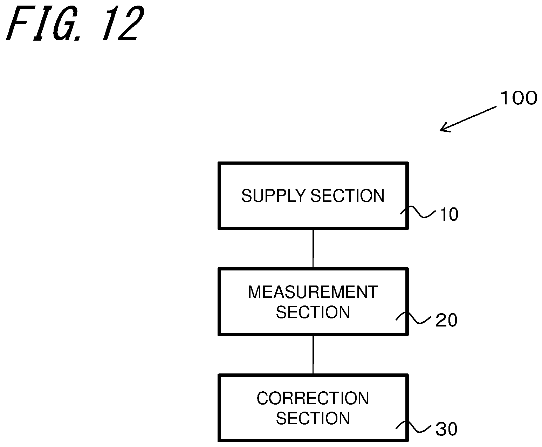

FIG. 12 is a block diagram showing the functional configuration of the measurement device 100 according to one embodiment of the invention. The measurement method according to one embodiment of the invention can be executed by the measurement device 100.

The measurement device 100 includes a biosensor device including a supply section 10 which supplies various liquids to a detecting section on an upper surface of which a first reaction substance is placed; a measurement section 20 which measures a first signal value and a second signal value; and a correction section 30 which corrects the first signal value with use of the second signal value, the measurement section 20 and the correction section 30 being connected to the biosensor device.

In the measurement device 100 according to this embodiment, the respective supply steps including the first supply step A2 and the second supply step A4 mentioned above, are carried out through repeated use of a single supply section 10. Likewise, the first measurement step A3 and second measurement step A4 mentioned above are carried out through repeated use of a single measurement section 20. The correction step A5 mentioned above is carried out in the correction section 30. As to electrical connection, for example, electrical connection between the supply section 10 and the measurement section 20, as well as between the measurement section 20 and the correction section 30, may be established either by wired connection using a signal cable, etc. or wireless connection using an antenna, etc.

While the supply section 10 may include a detecting section on which a first reaction substance is immobilized, a supply path for delivering a sample to the detecting section, and a pump for directing a sample flow into the supply path, a structure of the supply section 10 is not limited to this.

While the measurement section 20 may include a unit including a device that inputs a signal to the detecting section and acquires a predetermined signal value on the basis of signal output from the detecting section, a structure of the measurement section 20 is not limited to this.

While the correction section 30 may include an arithmetic unit including an arithmetic element for determining a sensitivity correction coefficient on the basis of the first signal value and the second signal value measured by the measurement section 20, or an arithmetic element-equipped arithmetic unit, a structure of the correction section 30 is not limited to this.

The following describes an example of a biosensor device used for the measurement device 100. The biosensor device constitutes the supply section, the measurement section, and the calculation section.

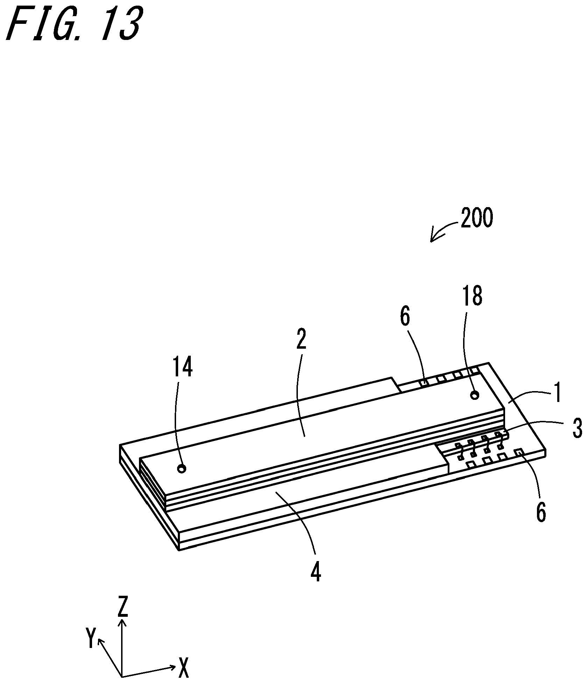

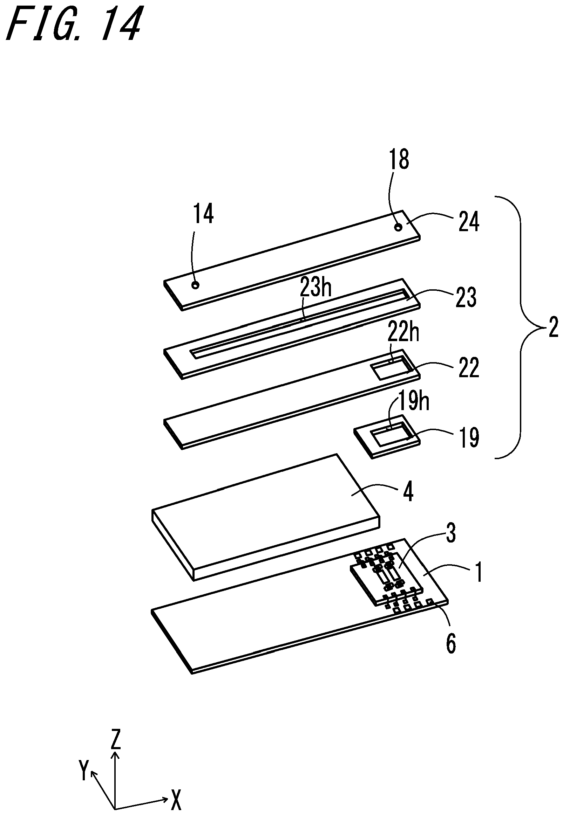

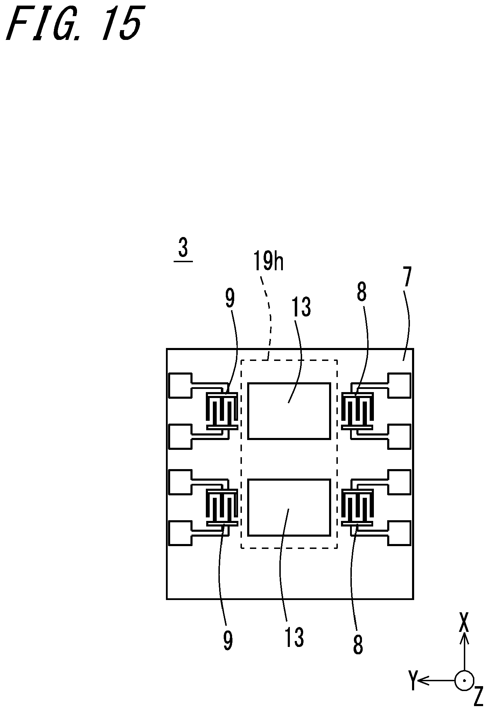

FIGS. 13 and 14 are a perspective view and an exploded perspective view, respectively, of a biosensor device 200, and FIG. 15 is a plan view of a detecting section 3.

The biosensor device 200 includes a substrate 1, a flow channel assembly 2, and a detecting section 3. As shown in FIG. 8, the flow channel assembly 2 is disposed on the substrate 1, with the detecting section 3 and a support member 4 lying in between. The flow channel assembly 2 includes an inlet 14 for entry of a sample in liquid form at a lengthwise one end part of the flow channel assembly 2, and a flow channel communicating with the inlet 14 is formed inside the flow channel assembly 2. The substrate 1 in the form of a flat plate is constructed of, for example, a resin substrate or a ceramic substrate, and includes a wiring conductor, etc. lying in the surface layer or inner layer thereof.

The detecting section 3 is mounted on one end part of the upper surface of the substrate 1. There are provided terminals 6 located on each of the opposite sides of the detecting section 3 so as to be electrically connected to the detecting section 3. The terminals 6 are connected with constituent devices, an arithmetic unit, etc.

The detecting section 3 is constructed of an elastic wave device and includes a piezoelectric substrate 7, a first IDT (Inter Digital Transducer) electrode 8, a second IDT electrode 9, and a reaction section 13. The piezoelectric substrate 7 is formed of a single crystal substrate having piezoelectric properties, such as a lithium tantalite substrate. The first IDT electrode 8 includes a pair of interdigitated-array electrodes. The interdigitated-array electrodes include two bus bars arranged facing each other, and a plurality of electrode fingers each extending from corresponding one of the bus bars toward the other. In the pair of interdigitated-array electrodes, the plurality of electrode fingers are arranged in an interdigitated pattern. The second IDT electrode 9 is structurally similar to the first IDT electrode 8. The first IDT electrode 8 and the second IDT electrode 9 constitute a transversal IDT electrode.

The first IDT electrode 8 serves to generate a predetermined elastic wave, and the second IDT electrode 9 serves to receive the elastic wave generated by the first IDT electrode 8. The first IDT electrode 8 and the second IDT electrode 9 are each made of, for example, aluminum or an alloy of aluminum and copper.

The reaction section 13 is disposed between the first IDT electrode 8 and the second IDT electrode 9. The reaction section 13 includes a two-layer structure including chromium and a film of gold formed on the chromium, for example. A first reaction substance is immobilized on the surface of the metallic film constituting the reaction section 13. When hemoglobin and a first correction substance are supplied onto the detecting section, they react with the first reaction substance immobilized on the surface.

Given that the first IDT electrode 8, the second IDT electrode 9, and the reaction section 13 make a set, the detecting section 3 is provided with two such sets. For example, the reaction section 13 of one of the sets serves for sample measurement, whereas the reaction section 13 of the other serves for reference value measurement. For example, hemoglobin is unreactive with the reaction section 13 of the other set.

In such a detecting section 3 utilizing elastic waves, to begin with, an external signal with a predetermined voltage is applied to the first IDT electrode 8. This causes the piezoelectric substrate 7 to be excited at the first IDT electrode 8, and an elastic wave of a predetermined frequency is produced. Part of the resulting elastic wave propagates toward the reaction section 13, passes through the reaction section 13, and is received by the second IDT electrode 9. In the reaction section 13, the first reaction substance reacts with hemoglobin depending on the amount of hemoglobin. Thus, the mass of the reaction section 13 increases by an amount corresponding to the reaction extent. As the phase of the elastic wave passing through the reaction section 13 changes with the increase in mass, a voltage corresponding to the change is developed in the second IDT electrode 9. The difference in phase between the signal applied to the first IDT electrode 8 and the signal outputted from the second IDT electrode 9 is measured as phase variation.

On the upper surface of the substrate 1, the support member 4 is further mounted, and the support member 4 supports the flow channel assembly 2. The flow channel assembly 2 is placed so as to cover at least part of the detecting section 3. For example, the flow channel assembly 2 includes a first adhesive layer 19, a first hydrophilic sheet 22, a second adhesive layer 23, and a second hydrophilic sheet 24.

The first adhesive layer 19 has the form of a frame including a through hole 19h, out of which part of the detecting section 3 is exposed. The first hydrophilic sheet 22 is laminated on the first adhesive layer 19. The first hydrophilic sheet 22 includes a through hole 22h which is analogous to the through hole 19h. The first adhesive layer 19 and the first hydrophilic sheet 22 are stacked together so that their through holes can be brought into communication with each other. The second adhesive layer 23 is laminated on the first hydrophilic sheet 22. The second adhesive layer 23 includes a longitudinally extending through hole 23h constituting a flow channel. One end of the through hole 23h extends to a point overlapping with the through hole 22h. The second hydrophilic sheet 24 is laminated on the second adhesive layer 23. The second hydrophilic sheet 24 is provided with an inlet 14 and an outlet 18, each in the form of a through hole, located on opposite ends, respectively, of the second hydrophilic sheet 24. The inlet 14 and the outlet 18 are formed at positions overlapping with the through hole 23h.

(Another embodiment)

In the measurement device 100 according to another embodiment,
the detecting section includes a first detecting section on an upper surface of which a first reaction substance which can react specifically with a detection substance is placed, and a second detecting section on an upper surface of which a second reaction substance which can react specifically with a correction substance is placed.

Moreover, the correction substance specifically can react at least with the second reaction substance, and the supply section supplies the detection substance and the correction substance in an order or simultaneously onto the first detecting section and the second detecting section.

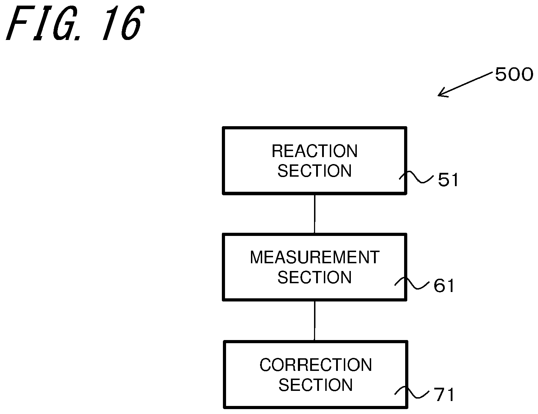

FIG. 16 is a block diagram showing the functional configuration of a measurement device 500 which is a modified example of the measurement device 100 according to another embodiment.

The measurement device 500 according to a modified example of another embodiment includes:

a first reaction section which reacts a detection substance with a first reaction substance which reacts specifically with the detection substance;
a second reaction section which reacts a correction substance with a second reaction substance which specifically reacts at least with the correction substance;
a measurement section which measures a first signal value derived from a reaction between the detection substance and the first reaction substance, and a second signal value derived from a reaction between the correction substance and the second reaction substance; and
a correction section which corrects the first signal value with use of the second signal value.

In this modified example, the measurement device 500 includes a biosensor device including a reaction section 51 which reacts a detection substance with a first reaction substance and reacts a correction substance with a second reaction substance; a measurement section 61 which measures a first signal value and a second signal value; and a correction section 71 which corrects the first signal value with use of the second signal value, the measurement section 61 and the correction section 71 being connected to the biosensor device. The reaction section 51, the measurement section 61, and the correction section 71 may be similar in constituent and material to the reaction section 13, the measurement section 20, and the correction section 30, respectively, as exemplified hereinabove.

In the measurement device 500 according to this embodiment, the first reaction step B1 and second reaction step B2 described above, as well as the first reaction step C1 and second reaction step C2 described above, are carried out through repeated use of a single reaction section 51. In this connection, the earlier described rules apply with respect to the supply section 61 and the measurement section 71.

As a matter of course, the invention is not limited to the embodiments and modified examples described heretofore, and thus improvements and changes may be made therein without departing from the scope of the invention.

Reference Signs List

| 1: | Substrate |
|----|-----------|
| 2: | Flow channel assembly |
| 3: | Detecting section |
| 4: | Support member |
| 6: | Terminal |
| 7: | Piezoelectric substrate |
| 8: | First IDT electrode |
| 9: | Second IDT electrode |
| 10: | Supply section |
| 13: | Reaction section |
| 14: | Inlet |
| 20: | Measurement section |
| 30: | Correction section |
| 100, 500: | Measurement device |
| 200: | Biosensor device |

**Claims**

1.  A measurement method, comprising:

    preparing a detecting section on an upper surface of a measurement device with a reaction substance that can react specifically with a detection substance and a correction substance, wherein the detecting section measures signal values based on reactions of the reaction substance;
    supplying the detection substance onto the detecting section;
    supplying the correction substance onto the detecting section;
    measuring a first signal value of the signal values based on a specific reaction between the detection substance and the reaction substance;
    measuring a second signal value of the signal values based on a specific reaction between the correction substance and the reaction substance; and
    correcting the first signal value using the second signal value.

2.  The measurement method according to claim 1,
    wherein the reaction substance can react specifically with each of the detection substance and the correction substance, and
    the supplying the correction substance and the measuring a second signal value are carried out prior to the the supplying the detection substance or subsequent to the measuring a first signal value.

3.  The measurement method according to claim 1 or 2,
    wherein an amount of the correction substance is a known amount, or an excessive amount relative to the reaction substance.

**4.** The measurement method according to any one of claims 1 to 3, further comprising:
removing the correction substance from the detecting section.

**5.** The measurement method according to claim 1,
wherein the detecting section comprises a first detecting section on the upper surface of which a first reaction substance which can react specifically with the detection substance is placed, and a second detecting section on the upper surface of which a second reaction substance which can react specifically with the correction substance is placed,
the correction substance specifically can react at least with the second reaction substance, and
the detection substance and the correction substance are supplied in an order or simultaneously onto the first detecting section and the second detecting section.

**6.** The measurement method according to claim 5,
wherein the second signal value is measured after a reaction between the correction substance and the second reaction substance reaches a level of saturation.

**7.** The measurement method according to claim 5 or 6,
wherein the first signal value is divided by the second signal value in the correction step.

**8.** The measurement method according to any one of claims 5 to 7,
wherein the detection substance is supplied simultaneously with the correction substance onto the first detecting section, and is then supplied simultaneously with the correction substance onto the second detecting section.

**9.** The measurement method according to any one of claims 5 to 8,
wherein the correction substance and the detection substance are supplied in an order or simultaneously to a part of the upper surface of a third detecting section which is free of the first reaction substance and the second reaction substance,
the measurement method further comprises measuring a third signal value of the signal values based on a condition of the upper surface of the third detecting section, and
the first signal value is corrected using the third signal value.

**10.** The measurement method according to claim 9,
wherein the detection substance is supplied simultaneously with the correction substance onto the third detecting section, is then supplied simultaneously with the correction substance onto the first detecting section, and is lastly supplied simultaneously with the correction substance onto the second detecting section.

**11.** The measurement method according to any one of claims 5 to 10,
wherein the correction substance reacts specifically with the detection substance and the second reaction substance.

**12.** The measurement method according to any one of claims 5 to 11,
wherein the correction substance reacts specifically with the second reaction substance.

**13.** The measurement method according to claim 12,
wherein, after mixing the detection substance and the correction substance, the supplying the detection substance and the supplying the correction substance are simultaneously carried out.

**14.** The measurement method according to any one of claims 1 to 13, further comprising:
supplying a blocking agent onto the detecting section subsequent to preparing the detecting section on the upper surface.

**15.** The measurement method according to any one of claims 1 to 14,
wherein, correcting the first signal value comprises calculating a sensitivity correction coefficient based on the second signal value, or based on the first signal value and the second signal value, and correcting the first signal value based on the calculated sensitivity correction coefficient.

**16.** The measurement method according to claim 15,
wherein the first signal value is corrected by an operation using the calculated sensitivity correction coefficient.

**17.** The measurement method according to any one of claims 1 to 16,
wherein the detecting section comprises an elastic wave device, and
the first signal value and the second signal value comprise a phase characteristic value of the elastic wave device.

**18.** The measurement method according to any one of claims 1 to 17,
wherein the first signal value and the second signal value comprise a value measured by a quartz crystal microbalance (QCM) sensor, a surface plasmon resonance (SPR) sensor, or a field effect transistor (FET) sensor.

**19.** A measurement method, comprising:

a first reaction step of reacting a detection substance with a first reaction substance which reacts specifically with each of the detection substance and a first correction substance;
a second reaction step of reacting the first correction substance with the first reaction substance;
a first measurement step of measuring a first signal value derived from a reaction between the detection substance and the first reaction substance;
a second measurement step of measuring a second signal value derived from a reaction between the first correction substance and the first reaction substance; and
a correction step of correcting the first signal value with use of the second signal value,
the second reaction step and the second measurement step being carried out prior to the first reaction step or subsequent to the first measurement step.

**20.** A measurement method, comprising:

a first reaction step of reacting a detection substance with a first reaction substance which reacts specifically with the detection substance;
a second reaction step of reacting a second reaction substance with a correction substance which specifically reacts at least with the second reaction substance;
a first measurement step of measuring a first signal value derived from a reaction between the detection substance and the first reaction substance;
a second measurement step of measuring a second signal value derived from a reaction between the second reaction substance and the correction substance; and
a correction step of correcting the first signal value with use of the second signal value.

**21.** A measurement device, comprising:

a detecting section on an upper surface of the measurement device on which a reaction substance that can react specifically with a detection substance and a correction substance is placed, and which can measure signal values based on reactions of the reaction substance;
a supply section which supplies the detection substance and the correction substance to the detecting section;
a measurement section which measures a first signal value of the signal values based on a reaction between the detection substance and the reaction substance, and a second signal value of the signal values based on a reaction between the correction substance and the reaction substance; and
a correction section which corrects the first signal value using the second signal value.

**22.** The measurement device according to claim 21,
wherein the reaction substance can react specifically with each of the detection substance and the correction substance, and
the measurement section measures the second signal value prior to or subsequent to the measurement of the first signal value.

**23.** The measurement device according to claim 21,
wherein the detecting section comprises a first detecting section on the upper surface on which a first reaction substance which can react specifically with the detection substance is placed, and a second detecting section on the upper surface on which a second reaction substance which can react specifically with the correction substance is placed,
the correction substance specifically can react at least with the second reaction substance, and
the supply section supplies the detection substance and the correction substance in an order or simultaneously onto the first detecting section and the second detecting section.

**24.** A measurement device, comprising:

a first reaction section which reacts a detection substance with a first reaction substance which reacts specifically with the detection substance;

a second reaction section which reacts a correction substance with a second reaction substance which specifically reacts at least with the correction substance;

a measurement section which measures a first signal value derived from a reaction between the detection substance and the first reaction substance, and a second signal value derived from a reaction between the correction substance and the second reaction substance; and

a correction section which corrects the first signal value with use of the second signal value.

# FIG. 1

```
┌──────────────────┐
│   PREPARATION    │   A1
│      STEP        │
└──────────────────┘
         │
┌──────────────────┐
│   FIRST SUPPLY   │   A21
│      STEP        │
└──────────────────┘
         │
┌──────────────────┐
│      FIRST       │
│   MEASUREMENT    │   A3
│      STEP        │
└──────────────────┘
         │
┌──────────────────┐
│  SECOND SUPPLY   │   A22
│      STEP        │
└──────────────────┘
         │
┌──────────────────┐
│     SECOND       │
│   MEASUREMENT    │   A4
│      STEP        │
└──────────────────┘
         │
┌──────────────────┐
│   CORRECTION     │   A5
│      STEP        │
└──────────────────┘
```

# FIG. 2

| | |
|---|---|
| FIRST REACTION STEP | **B1** |
| SECOND REACTION STEP | **B2** |
| FIRST MEASUREMENT STEP | **B3** |
| SECOND MEASUREMENT STEP | **B4** |
| CORRECTION STEP | **B5** |

# FIG. 3

# FIG. 4

# FIG. 5

| | |
|---|---|
| PREPARATION STEP | A1 |
| SUPPLY STEP | A2 |
| FIRST MEASUREMENT STEP | A3 |
| SECOND MEASUREMENT STEP | A4 |
| CORRECTION STEP | A5 |

*FIG. 6*

| | |
|---|---|
| FIRST REACTION STEP | C1 |
| SECOND REACTION STEP | C2 |
| FIRST MEASUREMENT STEP | C3 |
| SECOND MEASUREMENT STEP | C4 |
| CORRECTION STEP | C5 |

# FIG. 7

# FIG. 8

# FIG. 9

FIG. 10

# FIG. 11

# FIG. 12

100

| SUPPLY SECTION | 10 |

| MEASUREMENT SECTION | 20 |

| CORRECTION SECTION | 30 |

# FIG. 13

# FIG. 14

# FIG. 15

# FIG. 16

500

REACTION
SECTION
51

MEASUREMENT
SECTION
61

CORRECTION
SECTION
71

## INTERNATIONAL SEARCH REPORT

| International application No. |
|---|
| PCT/JP2019/002145 |

A. CLASSIFICATION OF SUBJECT MATTER
Int. Cl. G01N29/02(2006.01)i, G01N5/02(2006.01)i, G01N27/00(2006.01)i

According to International Patent Classification (IPC) or to both national classification and IPC

B. FIELDS SEARCHED

Minimum documentation searched (classification system followed by classification symbols)
Int. Cl. G01N29/02, G01N5/02, G01N27/00

Documentation searched other than minimum documentation to the extent that such documents are included in the fields searched
Published examined utility model applications of Japan    1922-1996
Published unexamined utility model applications of Japan  1971-2019
Registered utility model specifications of Japan          1996-2019
Published registered utility model applications of Japan  1994-2019

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2010-2219 A (AISIN SEIKI CO., LTD.) 07 January | 20, 24 |
| A | 2010, paragraphs [0023]-[0042], fig. 1 & US | 1-19, 21-23 |
| | 2009/0317900 A1, paragraphs [0023]-[0047], fig. | |
| | IA, IB | |
| A | WO 2016/194879 A1 (KYOCERA CORP.) 08 December | 1-24 |
| | 2016, & US 2018/0149646 A1 & EP 3306318 A1 & CN | |
| | 107615070 A | |
| A | JP 2017-9492 A (JAPAN RADIO CO., LTD.) 12 January | 1-24 |
| | 2017 (Family: none) | |
| A | JP 2015-127694 A (AISIN SEIKI CO., LTD.) 09 July | 1-24 |
| | 2015, & US 2015/0185213 A1 | |

☐ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28.03.2019 | 09.04.2019 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)